Europäisches Patentamt

(19) **European Patent Office**

**Office européen des brevets**

(11) Publication number: **0 180 384 B1**

(12) # EUROPEAN PATENT SPECIFICATION

(45) Date of publication of patent specification: **24.04.91**  (51) Int. Cl.⁵: **G01N 33/546, //G01N33/553**

(21) Application number: **85307513.3**

(22) Date of filing: **17.10.85**

(54) **Magnetically responsive reagent carrier and method of preparation.**

(30) Priority: **01.11.84 US 667514**

(43) Date of publication of application:
**07.05.86 Bulletin 86/19**

(45) Publication of the grant of the patent:
**24.04.91 Bulletin 91/17**

(84) Designated Contracting States:
**BE CH DE FR GB IT LI NL SE**

(56) References cited:
**FR-A- 2 454 098**
**US-A- 4 115 534**
**US-A- 4 169 804**
**US-A- 4 452 773**

(73) Proprietor: **TECHNICON INSTRUMENTS COR-
PORATION(a Delaware corporation)**
**511 Benedict Avenue**
**Tarrytown, New York 10591-6097(US)**

(72) Inventor: **Cohen, Beri**
**177 White Plains Road**
**Tarrytown, NY 10591(US)**
Inventor: **Wong, Tak K.**
**92 Filbert Street**
**Hamden, CT 06514(US)**
Inventor: **Hargitay, Bartholomew**
**15 Edna Street**
**White Plains, NY 10606(US)**

(74) Representative: **Wain, Christopher Paul et al**
**A.A. THORNTON & CO. Northumberland**
**House 303-306 High Holborn**
**London WC1V 7LE(GB)**

## Description

This invention relates to a magnetically responsive particulate reagent carrier and to its preparation. The carrier particles can be coupled to or otherwise associated with reactive groups, such as enzymes, haptens or antibodies, and used as carriers for analytical reagents, for example in performing assays.

Magnetically responsive or attractable particles have been produced by several methods which incorporate magnetite, ferrite, chromium oxide or nickel powders into matrices to which reagents can subsequently be coupled.

In one such method, a magnetically responsive powder is suspended in a solution of a polymer which is then gelled or precipitated to form a solid mass. The solid so obtained is ground or milled to obtain an insoluble powder in which ferromagnetic granules are immobilized. Examples are described in Robinson et al, Biotechnol. Bioeng. 15 :603 (1973) and Pourfarzaneh et al, Methods of Biochemical Analysis , 28 :281-3 (1982). The resulting particles are mostly oblong, jagged and of irregular size and shape, and so exhibit poor flow properties. In addition, the number and mass of magnetic granules in individual particles are uncontrolled. US-A-4169804 describes a similar method in which a magnetically responsive powder is mixed with an aqueous solution of a matrix-forming material and then injected into a heated oil bath to form cross-linked micro-particles.

Another method involves the deposition of polymeric coatings on ferromagnetic powders by coacervation; for example, by the deposition and subsequent cross-linking of albumin onto barium ferrite or magnetite. Examples are given in Ithakissios, et al, Clin. Chem ., 23 :2072 (1977) and Widder et al, Clin. Immunol. and Immunopathol ., 14 :395-400 (1979). Other approaches to polymer-coated particles use polymerization, e.g. amide and/or methacrylamide with crosslinkers in the presence of magnetite, in a two phase (water/oil) system. Also, magnetite particles themselves have been used as redox polymerization initiators (see Kronick et al, Science, 200:1074 (1978)).

The traditional methods for producing such particles have relied on the use of ferromagnetic powders. The particles generally exceed the critical size of a magnetic domain and, therefore, possess a substantial magnetic remanence. Remanence is the magnetic induction that remains in a material after removal of the magnetizing force. It is this property which causes such particles to remain aggregated, even in suspensions, after a magnetic field, to which they have been exposed, is removed. Such particles are often spontaneously aggregated by the ambient terrestrial magnetic field. Hence, these particles are unsuited for use in automated assay systems which measure reaction rates, rather than end points, since such measurements require the periodic resuspension and redispersion of the particles.

To avoid problems associated with magnetic remanence, several methods have been developed which use superparamagnetic, rather than ferromagnetic, materials for the particles. Superparamagnetic materials, such as "ferrofluids", exhibit a very high magnetization at saturation (over $10^{-4}$T(600 Gauss) and show no magnetic remanence in the absence of an external magnetic field. Examples of the production of such materials include the microencapsulation of oil-based ferrofluids (see Kakimi et al, US-A-4342739) and the polymerization of aldehydes in the presence of aqueous ferrofluids (Rembaum et al, J . Macromolecula r Science, Chemistry , A13 :603-632 (1979); Rembaum, US-A-4,267,234; Larson, et al, Biotechnology Letters 1 :501-506 (1979); and Molday, et al, Nature , 268 :437-438 (1977)). Another example is shown in US-A-4452773. However, due to their very small size, these particles exhibit a very slow magnetic response. Further, in some cases the aqueous systems are susceptible to oxidation, so that these particles must be prepared fresh for each use or stored under an inert atmosphere.

Another method of making superparamagnetic particles "entraps" colloidal magnetite (a water-based ferrofluid) into or onto gel particles by prolonged contact with Sepharose (trade mark) beads. However, these particles leak iron oxide which prevents them from being useful in optical density or absorption measurements (see Griffin & Mosbach, App. Biochem. and Biotech ., 6 :283-292 (1981)). These particles are thus unstable in that they do not retain permanently the iron oxide entrapped therein.

Another method of making superparamagnetic particles has been described in US-A-4297337. It provides a diluted array of superparamagnetic crystals in a glassy matrix. However, these particles must be kept extremely small, e.g. less than one micron in diameter, to prevent their high specific gravity causing rapid sedimentation. The magnetic force on such small particles is very weak and, therefore, the fast magnetic response necessary for automated analytical systems cannot be achieved.

Thus, in summary, although magnetically responsive carries particles have been prepared and used as reagent carries in immunoassay procedures, each has possessed certain undesirable characteristics.

We have now devised a superparamagnetic particulate reagent carrier material which is very useful in automated analysers, has excellent stability and very low magnetic remanence.

According to the present invention, there is provided a stable superparamagnetic reagent carrier material which is in the form of substantially spherical hydrated particles of a diameter from 0.5 to 500 $\mu$m, the particles comprising a matrix of a polymeric material hydrated by an aqueous medium, and a magnetically responsive substance having a diameter of from 5nm to 500nm, and being colloidally stably dispersed within said matrix and entrapped therein, said substance being essentially free in said hydrated particles to rotate within said matrix under the influence of a magnetic field and thermal motion resulting in a high magnetic permeability and low magnetic remanence.

The invention also includes a process for preparing such a material which comprises (a) incorporating by passive transport iron compounds into water-insoluble water-swellable substantially spherical particles of a polymeric matrix; (b) converting the iron compounds so-incorporated to insoluble iron oxides having a diameter of from 5nm to 500nm whereby a colloidally and stably dispersed magnetically responsive moiety is generated in situ within said polymeric matrix, said entrapped iron oxide being essentially free to rotate within said matrix under the influence of a magnetic field and thermal motion; and (c) washing the polymeric matrix to remove soluble by-products, the hydrated particles so formed having a diameter of from 0.5 to 500$\mu$m.

The magnetically responsive carrier particles of the present invention are useful particularly, but not exclusively, in automated, non-isotopic immunoassays. Their spherical shape and preferred smooth surface permit them to be pumped through long, narrow conduits, such as in continuous flow analyzers, without clogging. The particles can be caused to migrate freely in a liquid medium or to be immobilized by means of an inhomogeneous magnetic field. For example, when drawing them to the side or bottom of an optical cuvette, a clear supernatant is rapidly obtained for photometric analysis. In the absence of such a magnetic field, the particles are easy to disaggregate and disperse into suspension in the liquid medium, as they exhibit extremely low magnetic remanence. The ease of repeated magnetic sedimentation and resuspension allows for reaction rates, rather than just end points to be determined. Their small size and low relative specific gravity provide a very slow sedimentation rate. This, combined with their excellent chemical and physical stability, allows assay reactions to continue over an extended time period.

The particulate reagent carrier of the invention comprises particles or beads each formed of a water-insoluble matrix, e.g. a gel, swollen (i.e. hydrated) in an aqueous solution, and having colloidally dispersed therein superparamagnetic granules. Each hydrated particle has a diameter of 0.5 to 100 $\mu$m and preferably has a smooth, substantially spherical surface having an axial ratio lower than 4:1. Preferably, the superparamagnetic granules, e.g. iron oxides, are present in sufficient quantity to impart a specific gravity of between 1.2 and 2.7 to said hydrated particles.

In the process of the invention, the particles are prepared by passive transport incorporation of iron compounds into substantially spherical, water-swellable crosslinked polymeric matrices. The iron compounds so-incorporated are then converted in situ to their respective iron oxides. The iron oxides remain colloidally dispersed within the polymeric matrices and impart superparamagnetic properties to the resulting hydrated particles.

The term "colloidal" as used herein means a size range of 5 to 500 nm. Particles in this size range are generally unstable unless stabilized by electrostatic forces or by steric hindrance. In the present invention, steric hindrance is provided by the polymeric matrix surrounding the magnetic substance.

The term "magnetically responsive" as used herein refers to the capacity of the particles to migrate, relative to their surroundings, under the influence of an external inhomogeneous magnetic field.

The reagent carrier particles of the invention comprise a polymeric matrix, in which the magnetic substance is incorporated, the matrix being water-insoluble but swollen by an aqueous medium. In its swollen state, the matrix is permeable to soluble iron compounds, such as iron salts, or soluble compounds of nickel and chromium. In an aqueous medium close to neutral pH and at room temperature, the matrix is chemically and mechanically stable indefinitely. The matrix can tolerate extremes such as strong acids or bases for times sufficient to complete the fabrication process and also is resistant to boiling water for hours. The particles formed of the matrix are substantially spherical, preferably having an axial ratio less than 4:1, and preferably also having a smooth, non-ragged surface. They have a diameter of 0.5 to 500 $\mu$m, preferably 5 to 50 $\mu$m. The surface of the particle has functional groups which can be activated for coupling chemistry, to attach antibodies, antigens, haptens, enzymes and other reagents to the particle. Two parameters which have been found to be particularly important are the crosslink density of the matrix material, e.g. a gel, and the diameter of the particles. The proper choice of both determines to a large extent the performance of the overall particle.

First, the crosslink density, or the water regain, of the matrix material determines the amount of iron compounds that can be incorporated during particle preparation. The larger the water regain, because of lower crosslink density, the more iron compound is incorporated per particle. However, too weakly crosslinked matrices have inherent disadvantages. Due to the large "pores", large molecules present in the milieu can diffuse into the matrix, which may result in interferences, e.g. non-specific binding, when the matrix is used as a reagent carrier in an immunoassay. Also, such matrices, when swollen, are inherently soft and mechanically weak, easily deformed and/or abraded. During further processing, the temporarily harsh conditions of very low and high pH may cause breakage of some crosslinks, which in weakly crosslinked matrices may cause significant changes in the properties of the particles. For highly crosslinked materials, this change is insignificant. Matrix materials which show an initial water regain of 5 to 20 ml/g are particularly well suited as reagent carriers. Matrix materials of lower regain can also be used, but multiple loading of the magnetic material may be necessary, as described below.

The size of the particles is also important for satisfactory performance. The hydrodynamic drag on a sphere increases as the square of its diameter, while the magnetic pull, and also the gravitational force, acting on it increases as the cube of its diameter. Therefore, for fast migration within the surrounding liquid or reaction mixture, a large particle is desirable. On the other hand, to optimize the total binding surface available to couple reagents, small-sized particles are desired. Accordingly, to obtain particles which exhibit sufficient high mobility and sufficient surface area, and thus balance these requirements, particles should preferably have a diameter of from 3 to 60 $\mu$m, most preferably 20 to 40 $\mu$m, usually designated by the wetmesh size of -400.

Regarding the composition of the matrix material, several crosslinked hydrophilic polymers have been evaluated. Some were found to have suitable properties, as described, while others did not and were useless as reagent carriers. An example of a useful material is crosslinked polyacrylamide gel, such as Biogel®(BioRad Laboratories, Richmond, CA), in which the iron oxides formed predominantly inside the gel matrix and only insignificant amounts formed on the surface or between the particles. In contrast, the crosslinked polysaccharide known as Sephadex®(Pharmacia Fine Chemicals, Piscataway, NJ) for no obvious reason appears to exclude iron salts, i.e. when attempting to prepare particles as described below, the iron hydroxide and/or iron oxide predominantly form on the surface of the gel particles. (Biogel and Sephadex are trade marks.)

In the process of the invention, iron compounds are incorporated by passive transport along with water into spherical swellable, crosslinked polymeric matrices. The iron compounds are then converted in situ into their respective iron oxides, and the iron oxides remain colloidally dispersed within the polymeric matrix.

We use the term "swellable" herein to mean the property of the matrix material to absorb a fluid medium, usually aqueous, with or without an accompanying increase in volume.

In the process, dry gel particles are contacted with a solution or iron salts to cause the gel to swell and to introduce by passive transport the iron ions into the interior of the particles. Although it is not a necessary condition, the preferred solvent is water. Anhydrous iron chlorides as well as some non-ionic compounds, e.g. iron carbonyl, are also soluble in organic solvents and such solutions can in principle be used to "load" the particles. A variety of iron salts can be used as long as a concentrated solution can be prepared. Again, for reasons of convenience, the chlorides $FeCl_2$ and $FeCl_3$ are preferred.

The ratio of the ferrous to ferric salts is important. While the particles prepared with ferric ions alone show negligible magnetic properties, ferrous salts yield a product of considerable magnetic susceptibility. A ratio of ferric to ferrous of 2:1 yields a mixed oxide of the composition of magnetite $Fe_3O_4$. Ratios within the range of 2:1 to 1:2 all yield magnetically responsive products. Because of oxidation by air during processing, it is advantageous to use more ferrous salts than required by theory.

Ferric chloride in water and at room temperature undergoes rapid partial hydrolysis leading to super polycations which have increased size and also high positive charges. Both effects can cause reduced permeation of the salts into the polymeric network after short initial permeation. Consequently, uneven iron penetration is observed. Therefore, it is advantageous to suppress the formation of such less-diffusing species by the addition of salts which stabilize aqueous solutions of ferric ions. While a concentrated (1.6 molar) solution of $FeCl_3$ turns turbid in a few hours, addition of sodium sulfite in less than 0.2 molar ratio causes the solution to stay clear, albeit deep brown-red. The loading of the gel particles with iron salts in the presence of bisulfite yields an even and uniform product. Subsequently, in the preparation of the particles, this bisulfite is washed out.

Contacting of the mixed salt solution with the dry gel particles is done such that no intersticial liquid remains by the time the particles have swollen to their full capacity. The end-point of swelling is easily detected by the observation of a "sparkling" yellow appearance not unlike a projectionscreen.

The next step in the preparation is the conversion of the iron salts to their respective iron hydroxides within the particles. To this end, the pH is raised to over 7 by submerging the gel particles in an alkaline medium. In principle, NaOH, $Na_2CO_3$, gaseous ammonia or other bases can be used, but the preferred one is a chilled dilute (3%) ammonia solution. The ammonia is volatile and, therefore, its excess can easily be boiled off later in the preparation. After prolonged stirring, the supernatant is decanted and discarded. The particles are then washed with copious amounts of water and allowed to equilibrate, at each washing, for at least 30 minutes, until the final supernatant shows no alkalinity, e.g. has a pH of 7.6 or less.

In the next step, the mixed iron hydroxides incorporated and trapped in the gel particles are converted to oxides by heat treatment. The dark brown particles are resuspended in de-ionized water and boiled for an hour. It is during this period that the magnetic response develops, owing to dehydration of iron hydroxide within the polymer network to form iron oxide, predominantly $Fe_3O_4$. After this dehydration, the gel particles are thoroughly washed to remove all soluble materials. It can be observed through the microscope that very small amounts of iron oxide cling to the surface of the gel particles which can be removed in an ultrasonic field. Therefore, the gel particles are transferred onto a nylon mesh of about 20 $\mu$m openings. By gently raising and lowering this sieve in an ultrasonic bath filled with de-ionized water, the "exomagnetite" shakes loose and falls through the screen. After several minutes of sonication, particles are harvested which have a smooth shiny surface and are very responsive to an inhomogeneous magnetic field.

An alternative method of iron oxide precipitation uses an organic base. In this procedure, the saturated swollen gel particles are washed on a glass filter with 5% dimethylformamide(DMF) in acetone to remove the small amounts of iron solution wetting the surface of the gel. After a short period of air drying, the organic base 4-amino-methyl piperidine is added in aqueous solution to precipitate the iron oxides inside the particles. This method leads to particles which do not have any visible iron oxides on their surface and therefore do not need subsequent purifications by ultrasonic treatment. The clean particles are then air dried or kept as a slurry in a stoppered flask.

The magnetic response of the beads can be measured simply by weighing them in a standardized vial which can be placed on top of a strong permanent magnet. The difference in weight with and without the magnet in place is designated as "pull" and is compared with the pull observed with the same volume of finely ground magnetite or barium ferrite powder. Using the procedures described above, particles can easily be made which have a measured pull of at least 0.4 times that of barium ferrite and 0.20 times that of precipitated magnetite powder (Fisons Ltd., Leics. U.K.). Magnetic carrier particles with this kind of susceptibility are able to exhibit a migration rate of over 5 mm/second under the influence of a strong magnetic force in a medium of 0.001 Pa s (one centipoise) viscosity.

If an even higher magnetic mobility is desired, there are two techniques available to accomplish this. The first, termed "multiple loading", increases the magnetic force acting on each particle. The second, termed "roasting", decreases the hydrodynamic drag acting on the particles. Although the particles tend to become aggregated by the magnetic field, and aggregates move faster in the inhomogeneous magnetic field than do the individual particles, both techniques increase the rate at which the particles can be magnetically moved in the surrounding solution.

The magnetic properties of the particles, prepared as described above, can be enhanced by "multiple loading" with iron salts. For example, the particles, formed as described above, may be finally dehydrated by acetone followed by air drying or drying in a stream of nitrogen. These dry particles can be subjected again, one or more times, to the complete process, as described. The resulting magnetic susceptibility of the final particles is considerably higher and more so for a triple-loaded material. In this process of multiple loading, the diameter of the particle is kept constant (same hydrodynamic drag) but the magnetic response of each particle is increased by virtue of its increased iron oxide content. After multiple loading, the iron oxide may constitute 50% or more by weight of the particle.

Another method, referred to as "roasting", increases the magnetic-hydrodynamic mobility by decreasing the hydrodynamic drag at constant force acting on the particle. This is accomplished by introducing added crosslinks into the gel in the weakly swollen or unswollen state. For example, polyacrylamide eliminates ammonia on heating above 200° C to form imides. Both interchain and intrachain eliminations are possible (to diacrylimide bridges and/or to glutarimides, respectively), both tending to reduce the swelling capacity of the polymer. Intrachain elimination reduces the hydrophilicity and flexibility of the chains. Interchain elimination reduces the volume to which the gel particle expands on swelling. The result is a particle which is more mobile in a magnetic field than its precursor, because of reduced drag as a consequence of smaller diameter. If roasting is performed on particles not previously polished by sonication, the reaction causes fusion of the external iron oxide to the surface of the particles. The so-generated oxide-rich surface can then be used for the chemical activation of the particles for coupling. Reference is made to Example III, hereafter.

The particles of the invention can be associated with a variety of reactive groups to provide a reagent particularly, but not exclusively, useful in automated non-isotopic immunoassays. These particles carry reactive sites predominantly on their surface and can be caused either to migrate or to be immobilized at will by means of an inhomogeneous magnetic field. In the absence of such a magnetic field, these particles are easy to disperse and to resuspend in a surrounding medium because of their very low magnetic remanence and, by virtue of their small size and low relative specific gravity, their sedimentation rate is low.

While magnetically responsive reagent carriers are known in the art, none have exhibited features comparable with those of the particles of this invention, so as to be particularly useful in automated immunoassays. Because the particles have a spherical and preferably smooth surface, they can be pumped through long narrow tubes, e.g. having internal diameters of less than 1 mm, without clogging, as are required in continuous-flow analyzers such as disclosed in U.S.-A-4141687. These particles, when magnetically sedimented, leave a clear supernatant and, therefore, do not interfere with the colorimetric response of the assay. Further, because of their low magnetic remanence, in the absence of a magnetic field, the particles can be redispersed or resuspended in the supernatant by gentle shaking, permitting reaction rate (rather than just end point) determinations through repeated cycles of magnetic sedimentation and resuspension in the supernatant.

While a variety of reactive groups can be coupled to the surface of said particles, the primary interest lies in either catalytic moieties (e.g. enzymes) or immunologically active ligands such as antibodies, antigens or haptens.

The particles, when coupled to haptens, can be used advantageously in a one-step extraction of specific antibodies from a suspension, e.g. ascites fluids, by stirring them into the suspension and then drawing them out by an inhomogeneous magnetic field. This simple operation replaces, therefore, centrifugation or filtration followed by affinity chromatography.

The particles can also be advantageously used to recover specific chemicals (e.g. valuable or toxic ions) from suspensions containing other particulate solids, which precludes chromatographic separation. Any such chemicals can be recovered by coupling ligands to the particles of this invention which complex with these specific chemicals. Once complexed, these specific chemicals can be magnetically separated and retrieved.

The following working examples are given by way of illustration and describe experiments which were performed in developing the present invention. Standard commercially available reagent grade chemicals were used wherever possible.

## EXAMPLE I

This Example presents a step-by-step preparation of magnetically responsive particles in accordance with the invention, the particles being particularly useful for automated immunoassays.

A solution of 43.5 g of $FeCl_2.5\ H_2O$, 30.0 g of $FeCl_3.6\ H_2O$ and 4.5 g of $Na_2S_2O_5$ was prepared in 57 of de-ionized water. This solution was reddish brown and limpid (density 1.39), and contained about 37% solids; $Fe^{2+}:Fe^{3+} = 2:1$.

A 90 ml portion of this solution was added to 60 g of dry spherical polyacrylamide particles (Bio-Gel® P-4 Extra Fine, BioRad, supra) in a beaker and gently stirred for 30 minutes. The solution was heated slowly in a water bath to 55°C and continually stirred to ensure uniform rate of particle swelling. As the solution was taken up by the swelling particles, stirring became more difficult until finally the brown mass turned bright yellow, signaling that the intersticial liquid has vanished. Stirring was maintained until the particles were free flowing. Then, the beaker was removed from the bath and cooled to room temperature.

The swollen particles so-prepared were added to 900 ml of a 3% ammonium hydroxide solution chilled to below 5°C in a large glass beaker and continually stirred. Ice was added to maintain the temperature around 5°C. After 30 minutes, the supernatant was decanted. The particles, which had turned dark greenish-brown, were washed six times with de-ionized water, allowing each time about 30 minutes for equilibration, to remove the excess ammonia and ammonium chloride.

After washing, the solids were suspended in 1.5 liters of de-ionized water and the suspension was brought to a boil with frequent stirring. Boiling was maintained for 60 minutes. The brown color of the particles gradually turned to black. The magnetic properties had now developed, as could be seen by placing a strong magnet to the wall of the beaker. After boiling, the particles were washed again with water at room temperature to remove all soluble material. Decantation was facilitated by the magnetic properties of the particles. After several washes, the boiling cycle and wash cycle were repeated and the particles were dispensed into a glass cylinder closed off at its bottom by a nylon screen of 20 μm openings (Nytex-3).

The cylinder was lowered into an ultrasonic bath containing de-ionized water and, while sonicating, the cylinder was slowly raised and lowered, causing the water to pass in and out of the glass cylinder through the screen. Loose iron oxide which formed in small amounts (a few percent of total) on the surface of the particles fell through the screen and was visible by its dark color. Microscopic examination showed that the originally dull-surfaced brown particles had become shiny, uniformly brown and transparent.

The final particles were air dried and weighed. The increase of weight, due to colloidal iron oxide trapped in the gel matrix, amounted to 25% of the original dry particle, or approximately 20% of the final particle. The magnetic susceptibility was determined by measuring the weight increase of a standard volume of the particles in a strong inhomogeneous magnetic field. Compared with pure precipitated magnetic iron oxide (Fison's Ltd., supra), the carrier was 0.25 times as susceptible. Fully water swollen particles had a specific gravity of about 1.35.

For even higher magnetic susceptibility, the whole process was repeated several times. It was possible to increase the magnetic force by a factor of 2.8 by doubly loading and by 3.8 by triply loading the particles. The multiple loaded particles have a considerably higher specific gravity which manifests itself in a higher sedimentation rate.

EXAMPLE II

This Example illustrates a method of eliminating the formation of magnetic iron oxides on the particle surface. This is accomplished by washing traces of the iron salts from the surface of the particles, prior to treatment with base, with a solvent which does not extract the iron salts from the interior of the swollen particle.

A stock solution of iron salts was prepared from 400 g $FeCl_3.6\ H_2O$, 200 g $FeCl_2.4\ H_2O$ and 600 ml distilled water. An 80 ml portion of this solution was added to 10 g polyacrylamide particles (P-4, -400 mesh from BioRad, supra ) and heated to $50^{\circ}$ C for 30 minutes to swell the particles. The suspension was transferred to a sintered glass funnel of medium pore size and excess solution was removed by suction. The particles were then washed with 100 ml solution of 5% dimethylformamide (DMF) in acetone, followed by four washes with 50 ml of the same solution. Then 50 ml of an aqueous solution of 10% 4-aminomethyl piperidine were added. After stirring for ten minutes, the particles were washed three times with 100 ml 0.001M(N) NaOH solution and boiled in the last wash for 30 minutes. The particles were then washed again with distilled water and air dried. The surface of the particles, so prepared, was free of any adhering iron oxides. Thus, this method avoids the need for purification of the particles by sonication.

The carrier particles of Examples I and II exhibited no physical or chemical degradation over extended periods, i.e. greater than six months.

EXAMPLE III

The mobility in a surrounding liquid medium of the particles of the invention can be increased by reducing their diameter while maintaining their iron content. This Example demonstrates a method for introducing additional crosslinking in the gel particle in the unswollen state.

A 20 g portion of gel particles, prepared as described in Example II, was introduced into a 500 ml flask, to which 20 glass beads, 6 mm in diameter, were added. The flask was mounted on a rotary evaporator with the condenser removed and immersed in a silicon oil bath heated to above $250^{\circ}$ C for 2 hours, while maintaining slow rotation. The glass beads prevented the gel particles from clumping or aggregating. Ammonia evolved, indicating the formation of additional crosslinks in the gel particles. The resulting particles do not swell appreciably in aqueous solution. Their magnetic response under water, on a volumetric basis, is up to 4 times greater than the same particles which were not so-treated.

EXAMPLE IV

This Example demonstrates that the particles of the present invention exhibit a relatively high magnetic permeability with very low magnetic remanence.

The gel particles, prepared by Example I, were placed in a cylindrical tube. The tube was placed in a magnetic field of 3000 ampere-turns and the resulting magnetic induction was measured with a Bell gaussmeter. (The field applied was not strong enough to cause saturation.) When the generating current of the solenoid was shut off, the magnetic remanence was measured on the gaussmeter. The following table shows that the ratio of the remanent flux density (remanence) to the permeability (at 3000 ampere-turn field) is negligible in the case of the particles of this invention compared with the other particles tested. The comparison of the disclosed particles, when dry and when swollen, shows that the colloidal magnetite generated therein is essentially free to rotate when the gel is swollen, while partially hindered from rotation when the gel is dried.

| Sample | Mag. Flux Density (Gauss) $(10^{-4}T)$ | Relative Permeability | Remanence (Gauss) $(10^{-4}T)$ | Rem/Perm $(10^{-4}T)$ |
|---|---|---|---|---|
| Air | 470 | 1.0 | 0.0 | 0 |
| Steel (hardened) | 6200 | 13.19 | 400 | 30.3 |
| Steel | 6800 | 14.47 | 95 | 6.55 |
| Iron | 6800 | 14.47 | 57 | 3.94 |
| Magnetite powder | 950 | 2.02 | 75 | 37.13 |
| Barium Ferrite | 640 | 1.36 | 26.8 | 19.70 |
| Dry particle | 570 | 1.21 | 9 | 7.44 |
| Swollen particle | 510 | 1.09 | 1.5 | 1.37 |

EXAMPLE V

This Example demonstrates the immobilization of an enzyme on the surface of the particles of this invention in such a way that its enzymatic activity is maintained:

In a 50 ml flask, 1.5 grams of air dried, triply loaded gel particles were dispersed in 25 ml toluene containing 10 ml of acetone. Also, 30 mg of polyethylenimine (Dow-P1000) was added as a 2% solution in toluene. The 50ml flask containing the suspension was slowly rotated and gradually heated to 70°C. A gentle stream of nitrogen was used to gradually remove the acetone. As the polarity of the solvent diminishes, the polyethylenimine (PEI) adsorbs to the surface of the particles. The so-deposited PEI was then crosslinked with 3 mg of ethylenebromide at 65°C in two hours. The particles were then filtered off, washed with acetone and dried in a stream of nitrogen.

The activation of the surface was accomplished by treating 200 mg of the PEI-coated particles with a reagent consisting of 2 ml phosphate buffer (pH = 7.4) and 0.1 ml of 0.5g glutaraldehyde in 5 ml isopropyl alcohol (Baker reagent grade). After shaking the suspension for 45 minutes at room temperature, the supernatant was discarded and the particles washed twice with 1 ml each of phosphate buffer.

Coupling of beta-galactosidase to the activated particles was accomplished by dissolving 20 mg of the enzyme (Sigma G5635-750 U/mg) in 0.5 ml of the same buffer and shaking the solution with the activated particles at 2°C for 3-1/2 hours before storing for 18 hours at 2°C. The particles were then washed with copious amounts of cold buffer, until no enzymatic activity could be detected in the supernatant. The washed enzymatically active, magnetically responsive particles were stored in the same buffer at 4°C.

To determine if the coupled enzyme had maintained its activity, 2 mg particles were introduced into an optical cuvette having an optical path length of 7 mm and 250 µl of a substrate (ortho-nitro phenyl galactoside, 600 mg/l in pH = 7.5 phosphate buffer) were added. The particles were resuspended by shaking between successive readings. Prior to each reading of optical density at 420 nm wavelength, the reagent particles were drawn to the bottom of the cuvette by a permanent magnet. Readings were taken every 24 seconds. The color of ortho-nitrophenol developed rapidly. The initial rate of color development was greater than 300 mA/min. In the absence of reagent particles, no such colour developed.

## EXAMPLE VI

This Example demonstrates the immobilization of an antibody on the surfaces of particles of this invention.

A suspension of 170 mg of particles, as described in Example III, in 20 ml toluene was prepared and silanized with 5 ml glycidoxypropyltrimethoxy silane (Aldrich Chem. Co. #23,578-4) by refluxing overnight. The particles were then washed with toluene and subsequently with acetone and dried. The activated particles were shaken for 3 days at room temperature with 10 ml of a solution of 400 $\mu$l of a precipitated IgG fraction containing 35 mg/ml of goat-anti-rabbit antibody (Antibodies, Inc.), in phosphate buffer pH 8.0. The preparation was then washed three times with 10 ml each of the same buffer and subsequently reacted with 0.1% aqueous ethanolamine for 4 hours at room temperature for deactivation of unreacted epoxy groups on the particles. The particles were then washed three times with 10 ml portions of the same buffer and subjected to a thyroxine ($T_4$) assay protocol. In this assay, 50 $\mu$l of a suspension (25 mg/ml of the particles were incubated with 20 $\mu$l of sample (serum), 50 $\mu$l of $T_4$-conjugate ($T_4$-beta-galactosidase and 50 $\mu$l of first antibody (anti-$T_4$ rabbit antiserum) for 15 minutes at 37° C in an optical cuvette. The magnetic reagent was magnetically sedimented and the supernatant discarded. The wash cycle was repeated once again using 1 ml of buffer. The particles were then suspended in the enzyme substrate of 500 $\mu$l of an orthonitrophenyl galactoside and, after 10 minutes at 37° C, 500 $\mu$l of molar sodium carbonate were added. The suspended particles were magnetically sedimented to the bottom of the cuvette and the absorption of the supernatant was measured at 420 nm wavelength. The amount of $T_4$-beta-galactosidase bound to the particles was calculated and compared with a commercial product of similar configuration. Such commercial product was a cellulose-based solid phase sold by us under Part No. T01-1059. The fraction of the enzyme bound to the solid phase was comparable for both, i.e. between 34% and 35%. However, the non-specific binding (NSB) as determined in the absence of the first antibody was only a fraction of that found with the commercial product.

## Claims

1. A stable superparamagnetic reagent carrier material which is in the form of substantially spherical hydrated particles of a diameter from 0.5 to 500 $\mu$m, the particles comprising a matrix of a polymeric material hydrated by an aqueous medium, and a magnetically responsive substance having a diameter of from 5nm to 500nm, and being colloidally stably dispersed within said matrix and entrapped therein, said substance being essentially free in said hydrated particles to rotate within said matrix under the influence of a magnetic field and thermal motion, resulting in a high magnetic permeability and low magnetic remanence.

2. A carrier material according to claim 1, which has a magnetic response greater than 20% of the magnetic response of magnetite.

3. A carrier material according to claim 1 or 2, wherein said matrix comprises an organic polymeric material, and/or a crosslinked gel, and wherein the particles preferably have a smooth surface.

4. A carrier material according to claim 1,2 or 3, wherein said magnetically responsive substance comprises oxides of iron.

5. A carrier material according to any of claims 1 to 4, wherein said magnetically responsive substance is dispersed in the polymeric matrix in a quantity to impart a specific gravity of between 1.2 and 2.7 to said hydrated reagent carrier particles.

6. A carrier material according to any of claims 1 to 5, wherein the colloidally dispersed magnetically responsive substance comprises granules having a diameter smaller than 50 nm, and/or it constitutes at least 20% by weight of said reagent carrier material.

7. A carrier material according to any of claims 1 to 6, wherein said particles have an axial ratio of less than 4:1, and preferably have a diameter of between 0.5 and 100 $\mu$m when hydrated.

8. A carrier material according to any of claims 1 to 7, wherein a reagent is immobilized on the surface of said particles, the reagent preferably being an enzyme, or an antibody, or an antigen, or a hapten or another ligand.

9. A process for preparing a stable superparamagnetic particulate reagent carrier as defined in claim 1 which comprises (a) incorporating by passive transport iron compounds into water-insoluble water-swellable substantially spherical particles of a polymeric matrix; (b) converting the iron compounds so-incorporated to insoluble iron oxides having a diameter of from 5nm to 500nm whereby a colloidally and stably dispersed magnetically responsive moiety is generated in situ within said polymeric matrix, said entrapped iron oxide being essentially free to rotate within said matrix under the influence of a magnetic field and thermal motion; and (c) washing the polymeric matrix to remove soluble by-products, the hydrated particles so formed having a diameter of from 0.5 to 500$\mu$m.

10. A process according to claim 9, wherein said polymeric matrix is selected from polymers and copolymers of acrylamide and methacrylamide.

11. A process according to claim 9 or 10, wherein the step of incorporating said iron compounds comprises diffusing said iron compounds into said matrix, or introducing said polymeric matrix into a solution of iron compounds, to effect passive transport of said iron compounds through swelling of said polymeric matrix particles.

12. A process according to claim 9, 10 or 11, wherein said iron compounds are salts of divalent and/or trivalent iron.

13. A process according to any of claims 9 to 12, wherein the step of converting said iron salts comprises reacting said iron salts with a base to form iron hydroxides and subsequently converting said iron hydroxides to iron oxides, preferably by heating.

14. A process according to any of claims 9 to 13, including the step of washing said particles following conversion of said iron compounds to the iron oxides, to remove any soluble by-products contained in and/or adhering to said hydrated carrier particles, the said step of washing preferably including the step of ultrasonically washing said polymeric matrix.

15. A process according to any of claims 9 to 14, comprising the further step of heating the particles to permanently anchor to their surface any extraneous particulate reaction products adhering to said particles.

16. A process according to any of claims 9 to 15, including repeating steps (a) (b) and (c), so as to increase the concentration of said iron oxides in said polymeric matrix and thereby enhance the magnetic response of said particles.

17. A process according to any of claims 9 to 16, comprising the further step of bonding a reagent onto said polymeric matrix particles.

18. The use of a reagent carrier as claimed in any of claims 1 to 8 or as made by the process of claim 17, in an immunoassay procedure, or in a manufacturing process involving enzymatic reactions, or in an isolation or purification process utilizing chemical affinity.

**Revendications**

1. Matériau porteur-réactif superparamagnétique stable qui est sous la forme de particules hydratées de forme pratiquement sphérique d'un diamètre de 0,5 à 500 $\mu$m, les particules comprenant une matrice d'un matériau polymère hydraté par un milieu aqueux, et une substance sensible au magnétisme ayant un diamètre de 5 nm à 500 nm, et qui est dispersée d'une façon stable de manière colloïdale à l'intérieur de ladite matrice et enrobée dans celle-ci, ladite substance étant pratiquement libre dans les particules hydratées de tourner à l'intérieur de ladite matrice sous l'influence d'un champ magnétique et d'un mouvement d'origine thermique ce qui a pour résultat une perméabilité magnétique élevée et une rémanence magnétique basse.

2. Matériau porteur selon la revendication 1, qui a une sensibilité magnétique plus grande que 20 % de la sensibilité magnétique de la magnétite.

3. Matériau porteur selon la revendication 1 ou 2, dans lequel ladite matrice comprend un matériau polymère organique et/ou un gel réticulé et dans lequel d'une manière préférable les particules ont une surface lisse.

4. Matériau porteur selon la revendication 1, 2 ou 3, dans lequel ladite substance sensible au magnétisme comprend des oxydes de fer.

5. Matériau porteur selon l'une quelconque des revendications 1 à 4, dans lequel ladite substance sensible au magnétisme est dispersée dans la matrice polymère en une quantité telle qu'elle communique un poids volumique situé entre 1,2 et 2,7 aux dites particules de porteur-réactif hydraté.

6. Matériau porteur selon l'une quelconque des revendications 1 à 5, dans lequel la substance sensible au magnétisme dispersée d'une manière colloïdale comprend des granules ayant un diamètre plus petit que 50 nm, et/ou elle constitue au moins 20 % en poids du dit matériau porteurréactif.

7. Matériau porteur selon l'une quelconque des revendications 1 à 6, dans lequel les dites particules ont un ratio axial plus petit que 4/1, et d'une manière préférable ont un diamètre situé entre 0,5 et 100 μm lorsqu'elles sont hydratées.

8. Matériau porteur selon l'une quelconque des revendications 1 à 7, dans lequel un réactif est fixé sur la surface des dites particules, le réactif étant d'une manière préférable un enzyme, ou un anticorps, ou un antigène, ou un haptène, ou un autre ligand.

9. Processus de préparation d'un porteur-réactif sous forme de particules superparamagnétiques stables tel que défini à la revendication 1 qui comprend (a) l'incorporation par transport passif de composés à base de fer à l'intérieur de particules pratiquement sphériques insolubles dans l'eau et gonflant à l'eau d'une matrice polymère; (b) la conversion des composés à base de fer ainsi incorporés en oxydes de fer insolubles ayant un diamètre allant de 5 nm à 500 nm ce par quoi une partie sensible au magnétisme dispersée de façon stable et d'une manière colloïdale est produite ainsi in situ à l'intérieur de ladite matrice polymère, ledit oxyde de fer enrobé étant pratiquement libre de tourner à l'intérieur de ladite matrice sous l'influence d'un champ magnétique et d'un mouvement d'origine thermique; et (c) le lavage de la matrice polymère pour enlever les sous produits solubles, les particules hydratées ainsi formées ayant un diamètre allant de 0,5 à 500 μm.

10. Processus selon la revendication 9, dans lequel ladite matrice polymère est sélectionnée à partir de polymères et de copolymères d'acrylamide et de méthacrylamide.

11. Processus selon la revendication 9 ou 10, dans lequel l'étape d'incorporation des dits composés de fer comprend la diffusion des dits composés de fer à l'intérieur de ladite matrice, ou bien l'introduction de ladite matrice polymère à l'intérieur d'une solution de composés de fer, pour effectuer un transport passif des dits composés de fer au moyen du gonflement des dites particules de matrice polymère.

12. Processus selon la revendication 9, 10 ou 11, dans lequel les dits composés de fer sont des sels de fer bivalent et/ou trivalent.

13. Processus selon l'une quelconque des revendications 9 à 12, dans lequel l'étape de conversion des dits sels de fer comprend une réaction des dits sels de fer avec une base pour former des hydroxydes de fer et par la suite convertir les dits hydroxydes de fer en oxydes de fer, d'une manière préférable par chauffage.

14. Processus selon l'une quelconque des revendications 9 à 13, incluant l'étape de lavage des dites particules qui suit la conversion des dits composés de fer en oxydes de fer, pour retirer tous les sous produits solubles contenus dans et/ou adhérant aux dites particules porteuses hydratées, ladite étape de lavage incluant d'une manière préférable l'étape de lavage par procédé aux ultrasons de ladite matrice polymère.

15. Processus selon l'une quelconque des revendications 9 à 14, comprenant l'étape ultérieure de chauffage des particules pour ancrer de manière permanente à leur surface certains produits réactifs étrangers sous forme de particules adhérant aux dites particules.

16. Processus selon l'une quelconque des revendications 9 à 15, incluant la répétition des étapes (a) (b) et (c), de manière à augmenter la concentration des dits oxydes de fer dans ladite matrice polymère et par ce moyen à augmenter la sensibilité magnétique des dites particules.

17. Processus selon l'une quelconque des revendications 9 à 16 comprenant l'étape supplémentaire de fixation d'un réactif sur les dites particules de matrice polymère.

18. L'utilisation d'un porteur-réactif tel que revendiqué dans l'une quelconque des revendications 1 à 8 ou tel que fabriqué par le processus de la revendication 17, dans une procédure d'immuno-essai ou dans un processus de fabrication impliquant des réactions enzymatiques, ou dans un processus d'isolement ou de purification utilisant l'affinité chimique.

## Ansprüche

1. Stabiles superparamagnetisches Reagenzträgermaterial, das in der Form von im wesentlichen sphärischen hydrierten Partikeln eines Durchmessers von 0,5 bis 500 $\mu$m vorliegt, welche Partikel eine Matrix eines polymeren Materials umfassen, das in einem wäßrigen Medium hydriert ist, und einer magnetisch empfindlichen Substanz, die einen Durchmesser von 5 nm bis 500 nm aufweist und kolloidal stabil innerhalb der Matrix dispergiert und darin eingeschlossen ist, wobei die Substanz in den hydrierten Partikeln im wesentlichen frei ist, sich unter dem Einfluß eines Magnetfeldes und thermischer Bewegung innerhalb der Matrix zu drehen, woraus eine hohe magnetische Permeabilität und geringe magnetische Remanenz resultieren.

2. Trägermaterial nach Anspruch 1, welches ein magnetisches Ansprechen größer als 20% des magnetischen Ansprechens von Magnetit aufweist.

3. Trägermaterial nach Anspruch 1 oder 2, in welchem die Matrix ein organisches polymeres Material und/oder ein vernetztes Gel umfaßt und in welchem die Partikel vorzugsweise eine glatte Oberfläche aufweisen.

4. Trägermaterial nach Anspruch 1, 2 oder 3, in welchem die magnetisch empfindliche Substanz Oxide von Eisen umfaßt.

5. Trägermaterial nach einem der Ansprüche 1 bis 4, in welchem die magnetisch empfindliche Substanz in der polymeren Matrix in einer Menge dispergiert ist, um den hydrierten Reagenzträgerpartikeln eine volumenbezoge Masse zwischen 1,2 und 2,7 zu erteilen.

6. Trägermaterial nach einem der Ansprüche 1 bis 5, in welchem die kolloidal dispergierte, magnetisch empfindliche Substanz Körnchen umfaßt, die einen Durchmesser von weniger als 50 nm aufweisen, und/oder die zumindest 20 Gew.-% des Reagenzträgermaterials bildet.

7. Trägermaterial nach einem der Ansprüche 1 bis 6, in welchem die Partikel ein Achsenverhältnis von weniger als 4:1 aufweisen und vorzugsweise einen Durchmesser zwischen 0,5 und 100 $\mu$m, wenn sie hydriert sind.

8. Trägermaterial nach einem der Ansprüche 1 bis 7, in welchem ein Reagenz auf der Oberfläche der Partikel festgesetzt ist, welches Reagenz vorzugsweise ein Enzym oder ein Antikörper oder ein Antigen

EP 0 180 384 B1

oder ein Hapten oder ein anderer Ligand ist.

9.  Verfahren zum Herstellen eines stabilen superparamagnetischen Partikel-Reagenzträgers nach Anspruch 1, welches umfaßt (a) die Einbringung von Eisenverbindungen über passiven Transport in wasserunlösliche, wasserquellfähige im wesentlichen sphärische Partikel einer polymeren Matrix; (b) die Umsetzung der so eingebauten Eisenverbindungen in nicht lösliche Eisenoxide, die einen Durchmesser von 5 nm bis 500 nm aufweisen, wodurch ein kolloidal und stabil dispergierter, magnetisch empfindlicher Anteil in sito innerhalb der polymeren Matrix erzeugt wird, wobei die eingeschlossenen Eisenoxide im wesentlichen frei sind, sich innerhalb der Matrix unter dem Einfluß eines Magnetfeldes und thermischer Bewegung zu drehen; und (c) das Waschen der polymeren Matrix zur Entfernung löslicher Nebenprodukte, wobei die so gebildeten hydrierten Partikel einen Durchmesser von 0,5 bis 500 $\mu$m aufweisen.

10. Verfahren nach Anspruch 9, in welchem die polymere Matrix aus Polymeren und Kopolymeren von Acrylamid und Methacrylamid ausgewählt ist.

11. Verfahren nach Anspruch 9 oder 10, in welchem der Schritt des Einbringens der Eisenverbindungen das Diffundieren der Eisenverbindungen in die Matrix oder die Einbringung der polymeren Matrix in eine Lösung von Eisenverbindungen umfaßt, um den passiven Transport der Eisenverbindungen durch Quellen der Polymer-Matrixpartikel zu bewirken.

12. Verfahren nach Anspruch 9, 10 oder 11, in welchem die Eisenverbindungen Salze von zweiwertigem und/oder dreiwertigem Eisen sind.

13. Verfahren nach einem der Ansprüche 9 bis 12, in welchem der Schritt des Umsetzens der Eisensalze die Reaktion der Eisensalze mit einer Base zur Ausbildung von Eisenhydroxiden und die darauffolgende Umsetzung der Eisenhydroxide in Eisenoxide, vorzugsweise über Erwärmung, umfaßt.

14. Verfahren nach einem der Ansprüche 9 bis 13, aufweisend den Schritt des Waschens der Partikal, welcher der Umsetzung der Eisenverbindungen in die Eisenoxide folgt, um jedwede lösliche Nebenprodukte, die in den hydrierten Trägerpartikeln enthalten sind und/oder daran haften, zu entfernen, wobei der Schritt des Waschens vorzugsweise den Schritt einer Ultraschallwaschung der polymeren Matrix einschließt.

15. Verfahren nach einem der Ansprüche 9 bis 14, umfassend den weiteren Schritt der Erwärmung der Partikel, um an ihrer Oberfläche dauerhaft jedwede äußere Partikel-Reaktionsprodukte, die an den Partikeln haften, zu verankern.

16. Verfahren nach einem der Ansprüche 9 bis 15, umfassend die Wiederholung der Schritte (a), (b) und (c), um die Konzentration der Eisenoxide in der polymeren Matrix zu erhöhen und hierdurch das magnetische Ansprechen der Partikel zu verstärken.

17. Verfahren nach einem der Ansprüche 9 bis 16, aufweisend den weiteren Schritt des Bindens eines Reagenzes an die Polymer-Matrixpartikel.

18. Verwendung eines Reagenzträgers nach einem der Ansprüche 1 bis 8 oder hergestellt nach dem Verfahren des Anspruchs 17 in einem Immunoassay-Prozeß oder in einem Herstellungsprozeß, der enzymatische Reaktionen beinhaltet, oder in einem chemische Affinität ausnutzenden Trennungs- oder Reinigungsprozeß.

13